(19) Europäisches Patentamt
European Patent Office
Office européen des brevets

(11) EP 1 372 640 B1

(12) **EUROPEAN PATENT SPECIFICATION**

(45) Date of publication and mention of the grant of the patent:
**19.07.2006 Bulletin 2006/29**

(21) Application number: **02719902.5**

(22) Date of filing: **23.02.2002**

(51) Int Cl.:
***A61K 31/407*** *(2006.01)* ***A61P 25/00*** *(2006.01)*

(86) International application number:
**PCT/EP2002/001915**

(87) International publication number:
**WO 2002/072089 (19.09.2002 Gazette 2002/38)**

(54) **USE OF THIOLUTIN DIOXIDE AND ITS DERIVATIVES IN THE MANUFACTURE OF A MEDICAMENT FOR THE TREATMENT OF CNS DISORDERS**

VERWENDUNG VON THIOLUTIN DIOXIDE UND IHREN DERIVATEN ZUR HERSTELLUNG EINES ARZNEIMITTELS ZUR BEHANDLUNG VON ZNS-KRANKHEITEN

UTILISATION DE DIOXYDE DE THIOLUTINE ET DE SES DERIVES DANS LA FABRICATION D'UN MEDICAMENT DESTINE A TRAITER LES MALADIES DU CNS

(84) Designated Contracting States:
**AT BE CH CY DE DK ES FI FR GB GR IE IT LI LU MC NL PT SE TR**
Designated Extension States:
**AL LT LV MK RO SI**

(30) Priority: **09.03.2001 EP 01105959**

(43) Date of publication of application:
**02.01.2004 Bulletin 2004/01**

(73) Proprietor: **Sanofi-Aventis Deutschland GmbH**
**65929 Frankfurt am Main (DE)**

(72) Inventors:
- **EDER, Claudia**
  **65719 Hofheim (DE)**
- **KURZ, Michael**
  **65719 Hofheim (DE)**
- **WINK, Joachim**
  **63322 Rödermark (DE)**

(56) References cited:
**WO-A-96/32396** **WO-A-99/12543**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the use of thiolutin dioxide and its derivatives in the manufacture of a medicament for the treatment of CNS disorders.

Thiolutin is a known natural compound which is available commercially (Apin Chemicals, UK; CMS Speciality Chemicals, UK; Ubichem plc, UK). Thiolutin dioxide is also a known compound. One known process for the preparation of thiolutin dioxide is by oxidation of thiolutin with m-chlorperbenzoic acid (Yield 30 %) as described in Schachtner et al., J. Heterocycl. Chem., 1999, 161-175.

Thiolutin dioxide has previously been described as having medicinal properties. WO 99/12543 describes the use of thiolutin dioxide as an antineoplastic agent and WO 96/32396 describes the use of thiolutin dioxide as an antibacterial and antifungal agent.

It has now surprisingly been found that thiolutin dioxide is an effective inhibitor of neurolysin. Neurolysin belongs to the family of zinc-containing metalloproteases. It plays a likely role in the physiological inactivation of neurotensin, an endogenous antipsychotic agent. Thiolutin dioxide inhibits the neurotensin inactivation of neurolysin and is therefore useful in the treatment of neurodegenerative diseases such as Parkinson's and Alzheimer's. Thiolutin dioxide is also selective in that it does not block other zinc-containing metalloproteases such as enkephalinase or angiotensin converting enzymes.

The present invention accordingly relates to the use of a compound of formula I

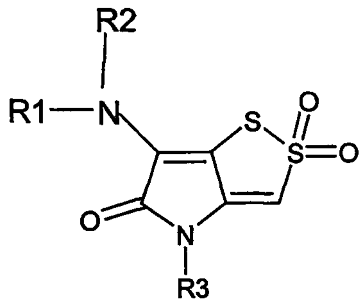

I

wherein

R1, R2 and R3 are independently selected from: H, alkyl, and acyl, and physiologically tolerated salts thereof, in the manufacture of a medicament for the treatment of CNS disorders.

Preference is given to compounds of formula I in which R1 is acyl, R2 is H and/or R3 is alkyl; and the physiologically tolerated salts thereof.

The acyl radicals in the compounds of formula I may have 2 to 10 carbon atoms, preferably 2 to 6 carbon atoms and can be straight-chain or branched, saturated or unsaturated once or twice.

An acyl radical with 2 carbon atoms means, for example, an acetyl radical.

Examples of saturated, unbranched acyl radicals are an acetic acid residue, propionic acid residue, butyric acid residue, valeric acid residue, caproic acid residue, enanthic acid residue, caprylic acid residue, pelargonic acid residue and capric acid.

Examples of unbranched acyl residues which are unsaturated once are an acrylic acid residue and crotonic acid residue.

An example of an unbranched acyl radical which is unsaturated twice is a sorbic acid residue.

The alkyl radicals in the compounds of formula I may have from 1 to 6 carbon atoms and can be straight-chain or branched. Further, the alkyl radicals include saturated as well as unsaturated groups which latter groups contain one or two double bonds. Examples of alkyl radicals containing from 1 to 6 carbon atoms are methyl, ethyl, propyl, butyl, pentyl and hexyl, the n-isomers of all these radicals, isopropyl, isobutyl, 1-methylbutyl, isopentyl, neopentyl, 2,2-dimethylbutyl, 2-methylpentyl, 3-methylpentyl and isohexyl.

Unsaturated alkyl radicals are, for example, alkenyl residues such as vinyl, 1-propenyl, 2-propenyl (=allyl), 2-butenyl, 3-butenyl, 2-methyl-2-butenyl, 3-methyl-2-butenyl,5-hexenyl or 1,3-pentadienyl.

In one embodiment, the invention relates to the use of a compound of formula II

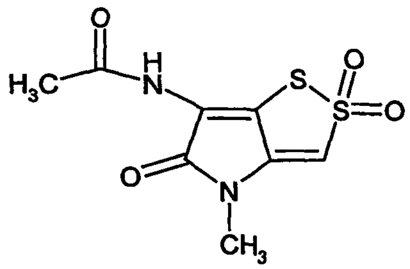

II

or a physiologically tolerated salt thereof in the manufacture of a medicament for the treatment of CNS disorders.

The compounds of formula I are useful in treating disorders caused by above normal levels of circulating neurolysin. CNS disorders which may be treated with the compounds of formula I include psychotic disorders such as schizophrenia and neurodegenerative disorders such as Alzheimer's and Parkinson's disease.

The compounds of formula I are obtainable by fermentation of the microorganism Nocardiopsis species ST 100692, DSM 13834, or one of its variants or mutants under suitable conditions, isolation of at least one compound, and conversion of the latter where appropriate into a physiologically tolerated salt, a derivative or a physiologically tolerated salt of the derivative.

In addition to producing thiolutin dioxide, Nocardiopsis species ST 100692, DSM 13834, also produces thiolutin under the conditions of fermentation described. The thiolutin obtained may be isolated and converted into thiolutin dioxide by methods known to the skilled man.

An alternative process for the production of a compound of formula I comprises cultivating the microorganism Nocardiopsis species ST 100692, DSM 13834, or mutants or variants thereof, in an aqueous nutrient medium, isolating and purifying thiolutin, converting thiolutin into at least one target compound and optionally further converting into a physiologically tolerated salt, a derivative or a physiologically tolerated salt of the derivative.

The microorganism Nocardiopsis species ST 100692 was deposited on 13 November 2000 under the conditions of the Budapest treaty at the Deutsche Sammlung von Mikroorganismen und Zellkulturen, Mascheroder weg 1 b, D-38124 Braunschweig under the number DSM 13834.

A taxonomic examination of the microorganism Nocardiopsis species ST 100692, DSM 13834, by analysis of fatty acids using gas chromatography showed the characteristic acids to be: 14:0 iso, 15:0 anteiso, 15:0 iso, 16:0, 16:0 iso, 17:0, 17:0 iso, 17:0 anteiso and 18:0. The colony colour is chrome yellow forming white aerial mycelium especially on ISP 2 (yeast-malt) and ISP 3 (oatmeal) medium.

In place of the strain DSM 13834 it is also possible to employ its mutants and variants so long as they synthesise the compounds according to the invention. Such mutants can be generated in a manner known per se by physical means, for example irradiation, such as, for example, ethyl methanesulfonate (EMS); 2-hydroxy-4-methoxybenzophenone (MOB) or N-methyl-N'-nitro-N-nitroguanidine (MNNG).

Screening for mutants and variants which produce the compounds according to the invention can take place by determining the biological activity of the active substances which have accumulated in the culture broth, for example by testing the neurolysin inhibition effect by the method described below.

Suitable and preferred as source of carbon for the aerobic fermentation are assimilable carbohydrates and sugar alcohols such as glucose, lactose or D-mannitol, and carbohydrate-containing natural products such as, for example, malt extract. Suitable nitrogen-containing nutrients are: amino acids, peptides and proteins, and their degradation products such as peptones or tryptones, also meat extracts, ground seeds, for example of com, wheat, soybean or the cotton plant, distillation residues from the production of alcohol, meat meals or yeast extracts, but also ammonium salts and nitrates. Inorganic salts which the nutrient solution my contain are, for example, chlorides, carbonates, sulphates or phosphates of the alkali metals or alkaline earth metals, iron, zinc, cobalt and manganese.

The formation of thiolutin dioxide is achieved particularly well for example in a nutrient medium which contains about 0.5 to 5% glucose, preferably 1 to 2 %, 0.5 to 5 % soybean meal, preferably 1 to 2 %, 0.1 to 1.5 % com steep (fluid), preferably 0.3 % to 0.8 %, 0.05 to 1.0 % calcium carbonate, preferably 0.1 to 0.5 % and 0.05 to 1 % sodium chloride, preferably 0.3 % to 0.8 %, in each case based on the weight of the complete nutrient solution.

The cultivation takes place aerobically, that is to say, for example, submerged with shaking or stirring in shaken flasks or fermenters, where appropriate with introduction of air or oxygen. The fermentation can be carried out, for example, in wide-necked bottles or round-bottomed flasks of various volumes, in glass fermenters or stainless steel tanks. It can be carried out in temperature range of about 20 to 35°C preferably at about 25 to 30°C. The pH should be between 4 and 10, advantageously between 6 and 8. The microorganism is cultivated under these conditions in general for a period of from 20 to 200 hours, preferably 24 to 150 hours.

Cultivation is advantageously carried out in several stages, i.e. one or more precultures are initially produced

in a liquid nutrient medium and are then transferred into the actual production medium, the main culture, for example in the ratio 1:10 by volume. The preculture is obtained, for example, by transferring a sporulated mycelium into the nutrient solution and allowing it to grow for about 20 to 120 hours, preferably 24 to 90 hours. The sporulated mycelium can be obtained, for example, by allowing the strain to grow for about 1 to 40 days, preferably 5 to 12 days, on a solid or liquid nutrient medium, for example yeast-malt agar or potato-dextrose agar.

The progress of the fermentation and the formation of the compounds according to the invention can be followed according to methods known to the skilled worker, such as, for example, by testing the biological activity in bioassays or by chromatographic methods such as thin-layer chromatography (TLC) or high performance liquid chromatography (HPLC).

The compounds of the formula I may occur both in the mycelium and in the culture filtrate, but the main amount is usually to be found in the biomass (mycelium). It is therefore expedient to separate the latter from the former by filtration or centrifugation. The filtrate is extracted with a water-immiscible solvent such as 1-butanol, ethyl acetate, chloroform or the like. The mycelium is expediently extracted with methanol or acetone, but it is also possible to use the above mentioned water-immiscible solvents.

The extractions can be carried out in a wide pH range, but it is expedient to operate in a neutral medium, preferably between pH 4 and pH 8. The organic extracts can, for example, be concentrated in vacuum and dried.

One purification method is chromatography on adsorption resins such as on Diaion® HP-20 (Mitsubishi Casei., Tokyo), on Amberlite ® XAD7 (Rohm and Haas, USA), and on Amberchrom® CG, (Toso Haas, Philadelphia, USA). Also suitable are numerous reverse phase supports, for example RP18, as have become generally known, for example, within the framework of high pressure liquid chromatography (HPLC).

The compounds of the formula I can be isolated and purified in analogy to the methods described above or by other methods known to the skilled man.

Derivatives of thiolutin dioxide are also covered by formula I above. These derivatives have the same effect or are converted under mild conditions into compounds having the same effect as thiolutin dioxide. The derivatives may be prepared by processes well known to the skilled man. Examples of preparative processes of some of the thiolutin dioxide derivatives covered by formula I are given below:

1) The (exocyclic) acetyl group of thiolutin dioxide can be cleaved with acid or base as described in the literature, e.g. in 'Protective Groups in Organic Synthesis', 3rd Edition, T. Greene & P. Wuts, John Wiley & Sons, 1999, pp 553-555.

2) The free amino group of thiolutin dioxide can be alkylated, for example, via reductive alkylation as described in 'Advanced Organic Chemistry', 4th Edition, J. March, John Wiley & Sons, 1992, pp 898-900.

3) The amino group of thiolutin dioxide can be acylated, e.g. with acid chlorides or anhydrides by standard procedures well known to one skilled in the art.

Further derivatives of compounds of formula I include those compounds which result by a reduction of at least one double bond of a compound of formula I, such as thiolutin dioxide, by methods given in the literature, for example, in P. N. Rylander, 'Hydrogenation Methods', Academic Press, New York (1985), Chpt. 2 or by dehydrohalogenation by methods described in H. O. House in 'Modern Synthetic Reactions', W.A. Benjymin, Inc., New York (1972), pp 446-452.

The compounds according to the present invention may be converted into pharmaceutically acceptable salts. The salts can be prepared by standard procedures known to one skilled in the art.

Physiologically tolerated salts of the compounds of the formula I mean both the organic and the inorganic salts thereof as described in Remington's Pharmaceutical Sciences (17th edition, page 1418 (1985)). Salts like sodium and potassium salts, for example, may be prepared by treating the compounds according to the invention with suitable sodium or potassium bases.

The compounds of the formula I may exist in various polymorphous forms, for example as amorphous and crystalline polymorphous forms. All polymorphous forms of the compounds of the formula I fall within the scope of the invention and are a further aspect of the invention.

The compounds according to the present invention and their pharmaceutically acceptable salts and derivatives can be administered to animals, preferably to mammals, and in particular to humans as pharmaceuticals on their own or in mixtures with one another.

Suitable pharmaceutical compositions accordingly comprise an effective amount of one or more of the compounds of the formula I or II, or pharmaceutically acceptable salts thereof, together with a pharmaceutically acceptable carrier.

The compounds according to the invention can be administered orally, intramuscularly, intravenously or by other modes of administration. Pharmaceutical compositions which contain these compounds or a pharmaceutically

acceptable salt or derivative thereof, optionally with other pharmaceutically active substances, can be prepared by mixing the active compounds with one or more pharmacologically tolerated auxiliaries and/or excipients. The mixture can then be converted into a suitable pharmaceutical form such as tablets, coated tablets, capsules, granules, powders, emulsions, suspensions or solutions.

Examples of auxiliaries and/or excipients which may be mentioned are fillers, emulsifiers, lubricants, masking flavours, colorants and buffer substances tragacanth, lactose, talc, agar-agar, polyglycols, ethanol and water. Suitable and preferred for parenteral administration are suspensions or solutions in water. It is also possible to administer the active substances as such, without vehicles or diluents, in a suitable form, for example, in capsules.

A method for the production of a suitable pharmaceutical comprises converting at least one of the compounds according to the present invention with a pharmaceutically suitable and physiologically tolerated carrier and, where appropriate, further suitable active substances, additives or excipients into a suitable dosage form.

As is customary, the galenic formulation and the method of administration as well as the dosage range which are suitable in a specific case depend on the species to be treated and on the state of the respective condition or disease, and can be optimised using methods known in the art. Using solid dosage forms, e.g. tablets or capsules, up to 500 mg, preferably 0.1 to 250 mg can be administered per day. For parenteral application up to 300 mg, preferably 0.5 to 150 mg, per day can be given.

The following are illustrative examples of the present invention but not limitative of the scope thereof:

Example 1: Production of a spore suspension of the producer strain Nocardiopsis species DSM 13834

100 ml of nutrient solution (4 g/l yeast extract, 15 g/l soluble starch, 1 g/l K2HP04, 0.5 g/l MgS04 x 7 H2O in 1000 ml water, pH before sterilization 7.0) in a 300 ml sterile Erlenmeyer flask were innoculated with the strain Nocardiopsis species DSM 13834 and incubated at 28°C and 180 rpm on a rotary shaker for 5 days. Subsequently, 1.5 ml of this culture was diluted with 1.5 ml 99 % glycerin and stored at -20 °C.

Example 2: Production of a culture or of a pre-culture of the producer strain in an Erlenmeyer flask

A sterile Erlenmeyer flask containing 100 ml of the following nutrient solution: 15 g/l soya flour, 15 g/l glucose, 5 g/l com steep fluid, 5 g/l NaCl and 2 g/lCaCo3, was innoculated with a culture which had grown in a slant tube (same nutrient solution but with 2 % agar) or with 1 ml of a glycerin culture (see Example 1) and incubated on a shaker at 180 rpm and 25 °C. The maximum production of thiolutin dioxide was reached after about 96 hours. A 72- hour old submerged culture (produced according to the process described for the shake culture but with the following medium: 15 g/l glucose, 15 g/l soya flour, 5 g/l com steep, 2 g/l CaCo3 and 5 g/l NaCl, pH 7.5) was sufficient for inoculating 10 and 100 l fermenters with an inoculum of about 5 %.

Example 3: Production of thiolutin dioxide

A 200 l fermenter was operated under the following conditions:

| | | |
|---|---|---|
| Nutrient medium: | Soya flour | 15 g/l |
| | Glucose | 15 g/l |
| | Com steep | 5 g/l |
| | NaCl | 5 g/l |
| | CaC03 | 2 g/l |
| | pH 7.2 (before sterilization) | |
| Incubation time: | 60 - 80 hours | |
| Incubation temperature: | 28 °C | |
| Stirrer speed: | 50 rpm | |
| Aeration: | 150 l/min | |

Foaming was suppressed by a repeated addition of a few drops of 1 to 2 ml of ethanolic polyol solution. The production maximum was reached after 69 hours.

Example 4: Isolation of thiolutin dioxide

3 l of the culture solution obtained in Example 3 were lyophilised and the lyophilisate was subsequently extracted

with methanol (2-3 l). The methanol extract was reduced in vacuum and diluted with water containing a methanol content of 10 %. The diluted extract was loaded onto a column with a capacity of 1 l packed with adsorption resin CHP-20P. Elution was with a solvent gradient from water to acetonitrile. Column through flow (30 ml/min) was carried out in fractions of 30 ml and the desired compound-containing fractions were collected, concentrated in vacuo and lyophilised to give approximately 30 mg of yellow-brown powder. The resultant powder was loaded onto a column packed with LUNA ® 10 uC18(2) (width x height = 21 mm x 250 mm) and eluted with a gradient of from 10 to 60 % acetonitrile in 0.1 % ammonium acetate/ water. The flow rate of the elution medium was 25 ml/min and outflow from the column was collected in fractions of 25 ml. Thiolutin dioxide was found in fractions 15 and 16. Lyophilisation of the named fractions gave 1.8 mg > 95 % pure thiolutin dioxide.

The physicochemical and spectroscopic properties of thiolutin dioxide can be summarised as follows:

Molecular formula: C8H8N204S2
Molecular weight: 260.3
UV-Maxima: 230, 302, 388 nm
1 H- and 13-C NMR: see Table 1

Table 1: 1 H- and 13-CNMR: Chemical shifts of thiolutin dioxide in DMSO at 300K

| | 1H | 13C |
|---|---|---|
| 1 | 2.10 | 22.51 |
| 2 | - | 170.46 |
| 3 | 10.65 | - |
| 4 | - | 123.01 |
| 5 | - | 164.27 |
| 6 | 3.10 | 27.85 |
| 7 | - | 145.48 |
| 8 | - | 114.05 |
| 9 | 7.55 | 109.58 |

Example 5: Thiolutin dioxide as neurolysin inhibitor.

An assay was performed on a CyBio pipetting system in a 384-well plate format in a final assay volume of 16 μl. In brief, 4 μl of appropriately diluted microbial extracts (dilution in 50 mM Tris buffer, pH 7.5) were distributed to the test plates (Greiner, white 384-small volume well plates). Thereafter, 4 μl of neurolysin (pre-diluted 1:5, 360 ng of protein) were added to each well. After a 10 minutes pre-incubation of the samples and the enzyme at room temperature the reaction was initiated by the addition of 8 μl of substrate (Mcc-Pro-Leu-Gly-D-Lys (Dnp)-OH, CALBIOCHEM) in Tris buffer. The final test concentration of the substrate was 4 μM.

After pipetting, the plates were immediately placed in a fluorometer (Spectra*Fluorplus*, SLT) and the initial amount of fluorescence was read (λex: 360 nm / λem 405 nm). The reaction was then allowed to proceed at 30°C for 30 min and a final fluorescence reading was taken.

The data are first blank corrected. Then, after subtracting the values at the time point zero from the respective values after 30 min, sample inhibition activity was expressed as

$$100 - (\text{Net Intensity compound} / \text{Net Intensity control}) \times 100\ (\%)$$

Each test plate contained a reasonable number of positive controls and blanks (buffer instead of enzyme).

The IC50 of thiolutin dioxide was found to be 0.6 μM

**Claims**

1. The use of a compound of formula I

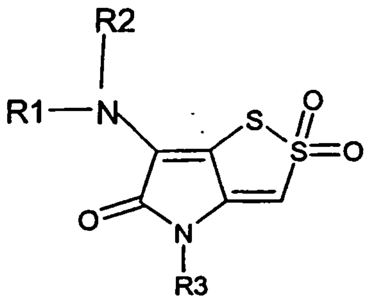

I

wherein
R1, R2 and R3 are independently selected from: H, alkyl, and acyl, and the physiologically tolerated salts and derivatives thereof, in the manufacture of a medicament for the treatment of CNS disorders.

2. The use of a compound of the formula I as claimed in claim 1 wherein R1 is acyl, R2 is H and R3 is alkyl.

3. The use according to claim 1 in which the compound is thiolutin dioxide of formula II

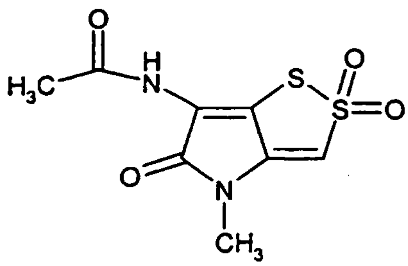

II

**Patentansprüche**

1. Verwendung einer Verbindung der Formel I

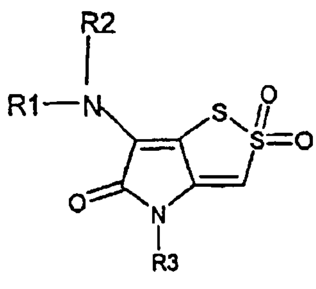

I

worin
R1, R2 und R3 unabhängig ausgewählt sind aus: H, Alkyl und Acyl, und den physiologisch tolerierten Salzen und Derivaten davon, zur Herstellung eines Arzneimittels für die Behandlung von Störungen des ZNS.

2. Verwendung einer Verbindung der Formel I nach Anspruch 1, worin R1 Acyl darstellt, R2 H darstellt und R3 Alkyl darstellt.

3. Verwendung nach Anspruch 1, worin die Verbindung Thiolutindioxid der Formel II

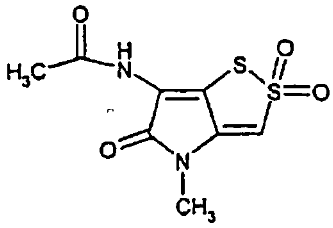

II

darstellt.

## Revendications

1. Utilisation d'un composé de formule I

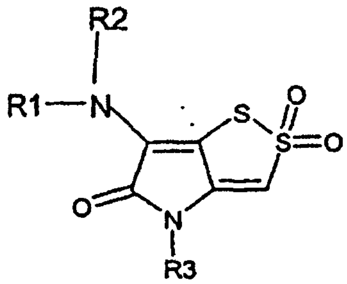

I

dans laquelle
R1, R2 et R3 sont choisis indépendamment parmi : H, alkyle et acyle, et les sels physiologiquement tolérés et les dérivés de celui-ci, dans la fabrication d'un médicament destiné au traitement de troubles du SNC.

2. Utilisation d'un composé de formule I selon la revendication 1, dans laquelle R1 est acyle, R2 est H et R3 est alkyle.

3. Utilisation selon la revendication 1, dans laquelle le composé est le dioxyde de thiolutine de formule II

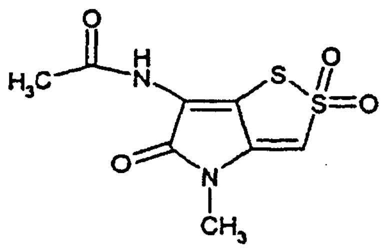

II